# EUROPEAN PATENT APPLICATION

(11) **EP 1 637 179 A1**
(43) Date of publication of application: **22.03.2006**
(21) Application number: 04255614.2
(22) Date of filing: 16.09.2004
(51) Int. Cl.: A61N 1/04, A61B 5/0408, A61B 19/02, B65D 77/20, B65D 77/26

(54) **Combination of an electrode storage and dispensing assembly and an electrode device**

(71) Applicant: Unomedical Limited, Redditch, Worcestershire B98 9N1 (GB)
(72) Inventor: Cummings, Edward Andrew, Armagh, Co. Armagh BT61 9EP (GB); Overbury, Bryan, Evesham, Worcestershire WR11 8QN (GB); Ledgeway, Andrew, Fleet, Hampshire GU52 6JG (GB); Wilcox, Alun, Richmond, Surrey TW10 7LQ (GB)
(74) Representative: Nielsen, Henrik Sten

(57) **Abstract**

A combination of a storage and dispensing assembly for storing and dispensing an electrode device and the electrode device arranged in the assembly. The electrode device comprises a sensor (18), a connector (20) for connecting the sensor to a monitoring or measuring means and an electrically conductive wire (22) for connecting the sensor to the connector. The assembly further comprises a container (14) for receiving the electrode device and having a dispensing aperture closed by a cover (12) attached to the container by means of first releasable attachment means. The sensor is releasably attached to a surface of the cover facing the interior of the container by means of second releasable attachment means.

## Description

The present invention relates to a combination of a storage and dispensing assembly for storing and dispensing an electrode. Furthermore, the present invention relates to a set of storage and dispensing assemblies for storing and dispensing a set of electrodes. Still further, the present invention relates to a method for providing storage and dispensing of an electrode from a storage and dispensing assembly and a method for providing storage and dispensing of a set of electrodes from a set of storage and dispensing assemblies.

Storage and dispensing devices relating to the present invention are some times know as blister packs, and have been described in patent publications such as US 5,390,792, US 2003/0168370, US 2003/0148110, US 6,358,241, US 6,183,467, US 6,478,030, US 5,814,043, US 2002/0104770 and US 2003/0014040. Reference is made to all the above-mentioned US patent publications, all of which are hereby incorporated in the present specification by reference in their entirety for all purposes.

A conventional electrode device, such as a disposable electrode device for use in a monitoring system or measuring apparatus, e.g. an ECG monitoring system comprises a sensor, an electrical wire and a connector for establishing electrically conductive connection to the monitoring or measuring apparatus. Generally, a problem exists in handling and properly storing the electrode devices and allowing an easy handling of the multiplicity of electrode devices without mixing the wires together and also keeping the wires or conductors of the individual electrode devices separate from one another without tying or mixing the conductors or wires together.

Conventionally, the electrode devices are stored in a state in which the sensor is fixated to a supporting foil such as a plastics foil and the wire or conductor of each of the electrode devices is wound up into a bundle. The handling of the individual electrode device in a monitoring or measuring situation may sometimes turn out to be inadequate and time consuming as the handling of the individual electrode device having the wire wound up in the bundles and the rewinding of the bundles before or after the electrode devices are applied in their intentional position on respective skin surface parts of a patient or person often tend to be tangled.

Therefor, there exists a need for providing a storage and dispensing assembly for storing and dispensing devices such as electrodes. According to a first aspect of the present invention, a combination of a storage and dispensing assembly for storing and dispensing an electrode is provided. The electrode being arranged in the assembly,
the electrode comprising an electrode head, a connecting means for connecting the sensor to a monitoring or measuring means and a electrically conductive wire for connecting the electrode head to the connector,
the assembly comprising a container for receiving the electrode and having a dispensing aperture closed by a cover attached to the container by means of first releasable attachment means, the electrode head being realisably attached to a surface of the cover facing the interior of the container by means of second releasable attachment means.

The electrode may be placed in a receiver, e.g. a mechanical device for holding the electrode in a fixed position inside the container. In a preferred embodiment of the present invention, the second releasable attachment means comprises re-sealable adhesive suited for attachment of the electrode to human skin.

The adhesive firstly adheres the electrode to the covering means. When the assembly is opened, the person opening the assembly may remove the electrode head and apply the electrode to the intended surface. The adhesive may then ensure that the electrode in adhered to that intended surface.

The intended surface may be the skin of a patient, such as an infant, a child, an adolescent, an adult or, alternatively an animal.

In one embodiment of the present invention, the electrically conductive wire may be a lead wire being transparent to radio waves.

A first feature of the present invention relates to the covering means comprising or being constituted by a flexible sheet and/or the first releasable attachment means being constituted by a peelable adhesive such that the flexible sheet may be manually peeled away from the container. The flexible covering means may be a PE, PP, PVC foil, aluminium foil, a combination thereof or PE-Al-PE laminate or any other laminate. Alternatively, the first releasable attachment means may be constituted by heat welding. The container may define an extending flange or area to which the cover may be attached, by e.g. heat welding or by means of an adhesive.

Alternatively, the covering means may be constituted by a rigid structure constituting a lid, such as a plastic lid.

According to a second feature of the present invention, the container may be made from a plastics material such as PE, PP or PVC, preferably being a transparent or translucent material, further preferably a material with a low moisture vapour transmission rate (MVTR). The transparent or translucent material allows a person to visually inspect the contents of the container. In the present context, the term person is to be construed as a generic term covering medical personnel, such as nurses and doctors, and any person that will apply an electrodes stored in an assembly according to the present invention onto an intended surface.

A third feature of the present invention relates to the electrode head comprising a woven, breathable cloth, a medical grade polyethylene foam and a sliver, silver chloride, tin chloride or graphite sensing element.

According to a fourth feature of the present invention, the electrode head may define a gripping part. The gripping part may be a part of e.g. the woven, breathable cloth or the polyethylene foam. Alternatively, any other mechanically stable part of the electrode head.

The gripping part is contemplated to facilitate a person applying one or more of the electrode heads to an intended surface to separate the electrode head from the covering means. The gripping part may be formed as a handle, handgrip, projection or protruding part.

A fifth feature of the present invention relates to the container defining an open-faced cavity and a flange extending outward from the cavity, a circumferential groove formed in the flange and the wire being received within the groove. The flange may extend from the container so that a plane is defined whereon the cover may be placed. The cover may be fastened to the flange or collar by means of adhesive, mechanical fastening, heat welding or the like, alternatively any combinations thereof.

According to a sixth feature of the present invention, a holder may be formed in the open-faced cavity for receiving the connector. The holder is contemplated to ensure that the connector is not moved out of its position within the container, e.g. during storage and/or transport. Also, when a person removes the cover, the wire is also removed from the container and the holder is contemplated to keep the connector in place until the electrode head have been positioned correctly on the intended surface, provided the person do not, intentionally or unintentionally, removes the connector from the container.

A seventh feature relates to the container defining a cavity, the wire being wound in a loop, the loop received in the cavity. The wire may be coiled or wound into a loop, which may then be placed into the cavity. This enables a machine or person to wind, spin, coil or loop the wire so that the wire may be received within the cavity. When the wire loop is then released inside the cavity, the wire is contemplated to unwind and press against the sidewalls of the cavity, thereby holding itself in place. Alternatively, the loop may be supported by a body, the body and the loop may then be received in the cavity. Supporting the wire loop with a body enables the wire to be tightly wound around the body, and possibly fixating the wire to the body so that the wire will not unwind after placing the body and wire in the cavity.

Advantageously, the body may define a circumferential groove wherein the wire is received. The risk of the wire then unwinding from the body during storage, transport or dispensing is contemplated to be reduced.

According to the teachings of the present invention, the container may define an open-faced cavity and a holder may be formed in the open-faced cavity for receiving the connector. The holder is contemplated to ensure that the connector does not exit the cavity during the application or dispensing of the electrode device.

According to a second aspect of the present invention, a set of storage and dispensing assemblies for storing and dispensing a set of electrodes is provided, such an electrode being arranged in each of the assemblies,
each of the electrodes comprising a sensor, a connecting means for connecting the sensor to a monitoring or measuring means and a electrically conductive wire for connecting the sensor to the connector,
each of the assemblies comprising a container for receiving a respective electrode and having a dispensing aperture closed by a cover attached to the container by means of first releasable attachment means, the sensor being realisably attached to a surface of the cover facing the interior of the container by means of second releasable attachment means.

Persons using the storage and dispensing devices according to the present invention may require placing a number of electrodes on the intended surface. The number of electrodes may be 2, 3, 4, 5 or even more. Therefor, the present invention provides the set of storage and dispensing devices according to the second aspect of the present invention. Each of the assemblies of the set of storage and dispensing assemblies may further comprises any of the features of the storage and dispensing device according to the first aspect of the present invention.

According to a third aspect of the present invention a method for providing storage and dispensing of an electrode from a storage and dispensing assembly is provided. The method according to the third aspect may comprise the steps of:
providing said electrode,
said electrode device comprising a sensor, a connector for connecting said sensor to a monitoring or measuring means and a electrically conductive wire for connecting said sensor to said connector,
providing an assembly, said assembly comprising a container for receiving said electrode device and having a dispensing aperture closed by a cover attached to said container by means of first releasable attachment means,
arranging said electrode device in said assembly, and
realisably attaching said sensor to a surface of said cover facing the interior of said container by means of second releasable attachment means.

It is a first object of the present invention that the method according to the third aspect, wherein the container defines an open faced cavity and a flange extending outward from the cavity, may further comprise the steps of:
providing a circumferential groove formed in the flange,
positioning the wire within the groove.

Providing a groove is contemplated to provide an orderly storage of the wire within the container. Also, it is contemplated that when applying the electrode to the intended surface, the wire will unravel in a orderly fashion, so that the wire does not get tangled or otherwise hindered.

A second object of the present invention relates to
providing a holder in the container, and
positioning the connector in the holder.

A holder is contemplated to provide an improved storage of the electrode connector during transport and/or storage. Also, during the application of the electrode to an intended surface, the connector is held within the container and it is contemplated that this will reduce the risk of damaging the connector unintentionally.

A third object of the present invention, relates to the method according to the third aspect, further comprising:
providing a gripping part in the cover for allowing a person to at least partly separate the cover from the container.

A gripping part or handle, is contemplated to provide an improved grip for a person removing the cover of the assembly.

A fourth object of the present invention relates to providing a gripping part in the sensor so as to allow a person to detach the sensor from the covering means.

A gripping part or handle is contemplated to provide an improved grip for a person removing the sensor from the covering means of the assembly, while the part of the sensor intended for being in facial contact with an intended surface is not touched and thereof the risk of possible contamination is contemplated to be reduced.

The devices used in connection with the method according to the third aspect of the present invention may incorporate any of the features in connection with the first aspect relating to a storage and dispensing device.

A fourth aspect of the present invention provides a method for providing storage and dispensing of a set of electrodes from a set of storage and dispensing assemblies, that may comprise the steps of:
arranging each of the electrode devices in a specific assembly,
each of the electrode devices comprising a sensor, a connector for connecting the sensor to a monitoring or measuring means and an electrically conductive wire for connecting the sensor to the connector,
each of the assemblies comprising a container for receiving the electrode device and having a dispensing aperture closed by a covering means attached to the container by means of first releasable attachment means,
realisably attaching each of the sensors to a surface of a specific cover facing the interior of a specific container by means of second releasable attachment means.

After providing a plurality of storage and dispensing devices, sensors including an electrode head, a wire and a connector may be arranged in the assembly.

A seventh feature of the present invention relates to the covering means being constituted by a set of covers, where each cover seals or closes a specific container. The method may then further comprise removing a specific cover of the set for exposing a specific sensor and/or container. The cover may be removed from a particular container for extracting or removing a specific sensor stored in that particular container.

An eight feature of the method according to the present invention relates to the covering means being constituted by a unitary covering means. The cover may be constituted by a single piece of material, such as a foil or laminate, covering the entire set of assemblies. The containers may as well be connected so that a person applying electrodes to intended surfaces may hold one package comprising the desired number of electrodes at once. The set of assemblies may alternatively be separated by a health care person into individual assemblies.

According to a ninth feature of the present invention, the method may comprise the additional step of removing a specific cover for exposing a single specific electrode or removing the cover for exposing the set of electrodes.

The cover may be crafted so that it is possible for a person to remove the cover from a specific container without exposing the remaining containers, this may be achieved by, e.g. peeling the cover in a first direction. A person may alternatively remove the entire cover in one operation, e.g. by peeling the covering means in a second direction, different from the first direction, so that all of the containers are exposed, substantially at once.

The method according to the fourth aspect of the present invention may incorporate any of the features relating to the first, second or third aspect.

The present invention is now to be described in more detail with reference to the drawings, in which:
Fig. 1 is a schematic elevated view of a storage and dispensing assembly,
Fig. 2 is a schematic view of an electrode device separated from an assembly,
Fig. 3 is a schematic view of an assembly line for assembling storage and dispensing assemblies,
Fig. 4 is a schematic top view of a pack comprising three storage and dispensing assemblies,
Fig. 5 is a schematic view of a body supporting a wire loop, and
Fig. 6 are schematic illustrations of a process of winding a wire into a loop.

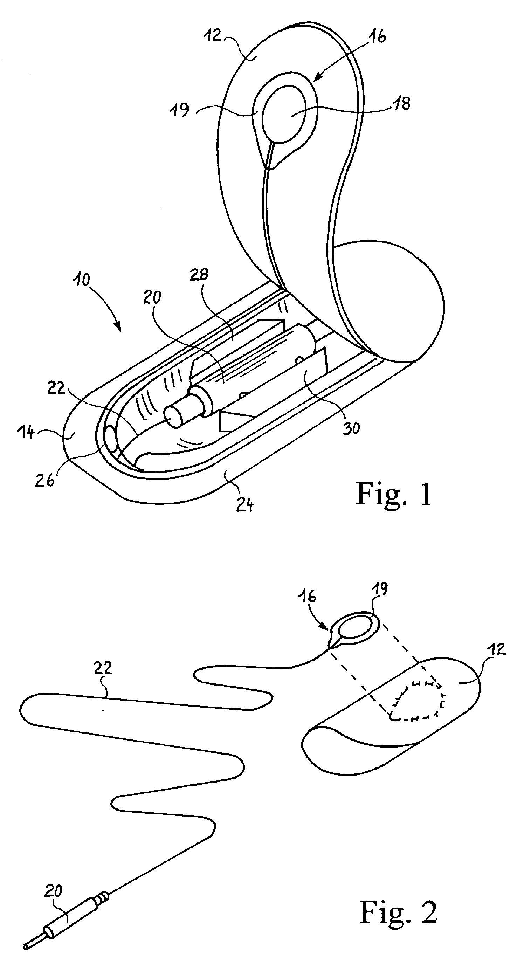

Fig. 1 is a schematic elevated view of a combination of a storage and dispensing assembly for storing dispensing an electrode device. The storage and dispensing assembly is in its entirety designated the reference numeral 10. The assembly 10 comprises a cover 12, preferably being a flexible plastics material foil or a flexible aluminium foil. The cover 12 is attached to a container 14. The cover is preferably heat sealed to the container 14 and may be peeled from the container 14.

An electrode device 16 is arranged inside the assembly 10, preferably fixated using a releasable adhesive so that a person, such as a nurse, doctor or other health care personnel, may remove the electrode device 16 from the cover 12 and position the electrode device 16 on an intended surface, such as a skin part of a patient. The electrode device 16 comprises a sensor 18, positioned in a sensor head 19. The sensor 18 preferably being an electrically conductive element, such as a sliver, silver chloride, tin chloride or graphite sensing element, a connector 20 for establishing a connection to a monitoring or measuring apparatus or system, and an electrically conductive wire 22 for establishing an electrical connection between the sensor 18 and the connector 20.

The container 14 defines a cavity wherein the connector 20, the wire 22 and the sensor head 19 is received. The container 14 further defines a flange 24 extending outwardly from the container 14. In the flange 24 a circumferential groove 26 is defined. The wire 22 or a part hereof, is preferably arranged within the groove 26 so that when a health care person applies the electrode device 16 to a patient, or a skin part of a patient, the wire 22 does not get tangled.

The connector 20 may be received within a holder, in the presently preferred embodiment of the present invention constituted by two protruding parts 28 and 30 for fixating the connector 20 during storage and/or transport and also while a health care person applies the electrode device 16 to the intended surface.

Fig. 2 is a schematic view of the electrode device 16 separated from the assembly 10 and the sensor head 19 detached from the cover 12.

In the presently preferred embodiment of the present invention, the sensor head 19 comprises two central fabric bodies, whereon a sensor electrode is mounted. The sensor 16 is applied with an adhesive, preferably an electrically conductive hydrogel or other skin-friendly adhesive. A lead wire assembly establishes the electrical connection from the sensor to the monitoring or measuring or apparatus. A foam cover may be applied on top of the lead wire and an upper fabric body.

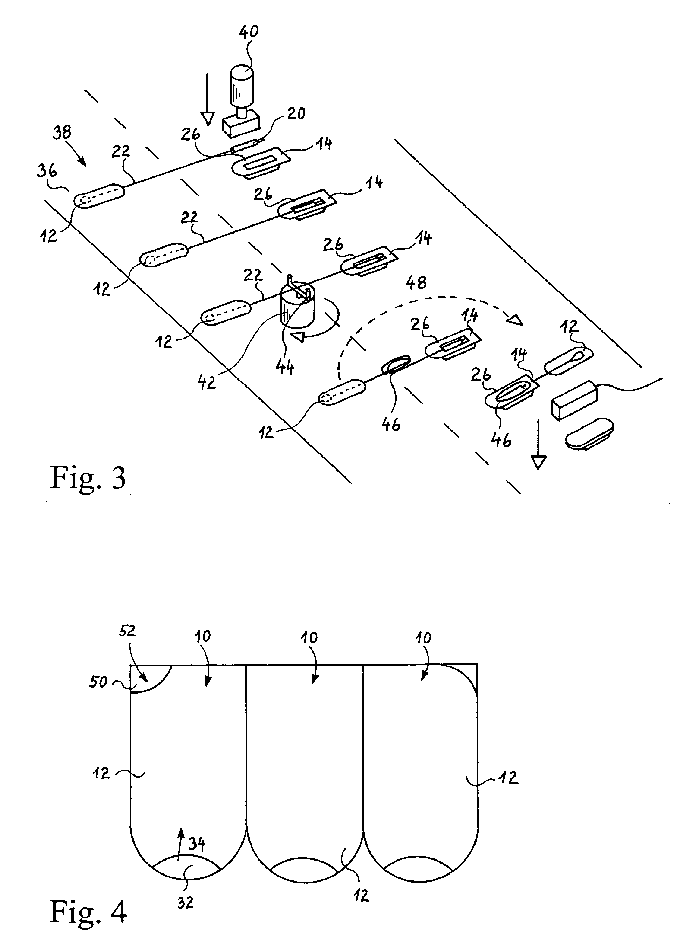

Fig. 3 is a schematic illustration of an assembly line for assembling covers and containers 14. The covers travel on a conveyor 36 in the direction of the arrow 38. The conveyor may include a laminate whereon the individual covers are formed e.g. by cutting or stamping a laminate. Also adhesive for adhering the covers 12 to the containers 14 may be applied to the cover 12.

The resealable and skin friendly adhesive is applied to either the electrode device 16 or to the cover 12 for fixating the sensor head 19 to the inside of the cover 12. The machine 40 places the connector 20 in the container 14, specifically between the protruding parts 28 and 30 in the cavity of a restrictive container. Prior to this, the container 14 may be stamped from a substrate by a stamping machine, thereby forming the individual parts of the container 14, such as the cavity, the flange 24, the groove 26 and the protruding parts 28 and 30.

A tool 42 for winding the wire 22 into a loop is also placed on or near the conveyor 36. The tool 42 preferably includes a rotational part 44 that grips the wire 22 and winds the wire 22 into a loop 46. Afterwards, the looped wire 46 is positioned in the cavity of the container, specifically and preferably into the groove 26 of the container 14. This may be achieved by moving the cover 12 in the direction of the arrow 48 so as to place or position the loop 46 substantially above or in the groove 26. Finally, the cover 12 may be positioned on the flange 24 and fixated to the flange, e.g. by heat welding or by an adhesive.

The order of steps performed on the assembly line is not to be considered limited to the order described in the present description. It might be contemplated to be advantageous to perform certain steps before or after other steps, for instance, the winding of the wire 22 may be performed during the positioning of the wire 22 in the container 14 or the groove 26, and the positioning of the connector 20 in the holder in the container 14 may be performed after the winding of the wire 22.

The covers 12 may be cut or stamped from a substrate or laminate at any point, before, after or during positioning of the electrode device 16.

Fig. 4 is a schematic top view of three assemblies supplied as one package. A health care person is thereby able to apply three electrodes to an intended surface without having to leave the patient for requiring more electrodes.

Even though Fig. 4 illustrates a package with three assemblies, two, four or even more assemblies may be assembled into a single package.

When the health care person is to apply the individual electrode devices 16, the health care person may desire to expose only one electrode device at one time or all of the electrode devices in the package. The health care person may choose to remove a single cover 12 by pulling the cover at the area designated 32 in the direction of the arrow 34. Alternatively, the health care person may pull the cover in the area 50 in the general direction of the arrow 52, thereby exposing multiple electrode devices at one time.

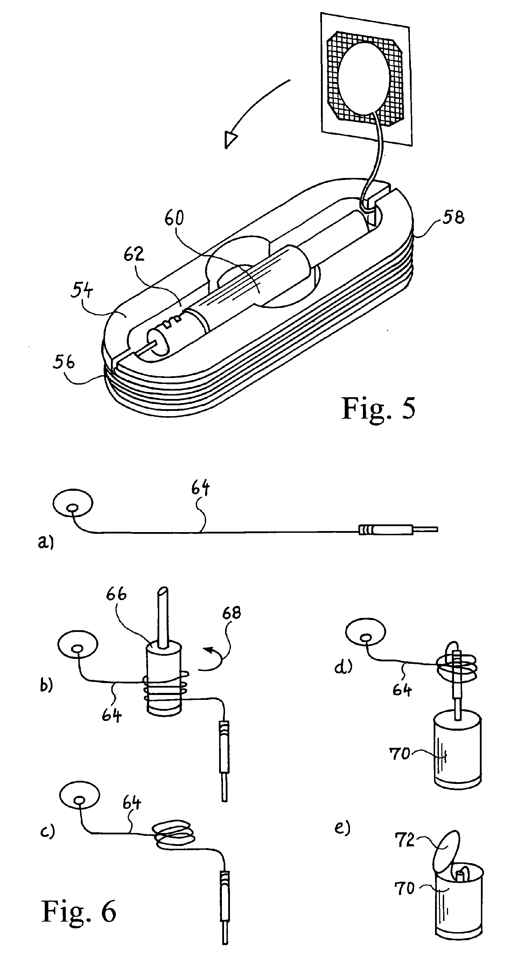

Fig. 5 is a schematic view of a body 54 supporting a wire loop 56. A wire is wound into a loop 56 for facilitating the positioning of the wire in the cavity of a dispensing assembly.

In the embodiment illustrated in Fig. 5, the wire loop 56 is received in a groove 58 defined in the body 54.

The body 54 illustrated in Fig. 5 is large enough to receive a connector 60 in a holder 62 defined in the body 54. However, embodiments where a connector 60 is not received in a holder in the body is also possible. Further more, the connector may be received in an aperture defined in the body.

Fig. 6 is schematic illustrations of a process of winding a wire 64 into a loop.

In a) the unwound wire 64 is illustrated. A tool 66 grabs the wire 64 at a point, preferably near or at the centre or middle of the wire 64. The tool 66 may then roll or wind the wire 66 into a loop, e.g. by rotating in the direction of the arrow 68.

The tool 66 may be removed, provided the loop may be maintained despite the lack of support, if it is not possible to remove the tool 66 without causing the loop to unwind, the tool 66 is not removed, skipping step c).

The wound wire 64 may then be placed or received in an appropriate container 70, as illustrated in d).

The wire loop may be supported by a body, such as that illustrated in Fig. 5, or the wire loop may be unsupported.

Finally, the container 70 may be sealed or closed by e.g. a lid 72, possibly constituted by a flexible cover, as discussed in relation with Figs. 1-4.

## Claims

1. A combination of a storage and dispensing assembly for storing and dispensing an electrode device and said electrode device arranged in said assembly,
said electrode device comprising a sensor, a connector for connecting said sensor to a monitoring or measuring means and an electrically conductive wire for connecting said sensor to said connector, and
said assembly comprising a container for receiving said electrode device and having a dispensing aperture closed by a cover attached to said container by means of first releasable attachment means, said sensor being realisably attached to a surface of said cover facing the interior of said container by means of second releasable attachment means.

2. The assembly according to claim 1, wherein said container defines a cavity and a flange extending outward from said cavity, said flange including a circumferential groove, in which said wire is received.

3. The assembly according to claim 2, wherein a holder is formed within said cavity of said container, in which holder said connector is received.

4. The assembly according to claim 1, wherein said container defines a cavity, said wire being wound in a loop, said loop received in said cavity.

5. The assembly according to claim 4, wherein said loop being supported by a body, said body and said loop received in said cavity.

6. The assembly according to claim 5, wherein said body defines a circumferential groove wherein said wire is received.

7. The assembly according to any of the claims 4-6, wherein a holder is formed within said cavity of said container, in which holder said connector is received.

8. The assembly according to any of the claims 1-7, wherein said second releasable attachment means comprises a re-sealable adhesive preferably a skin friendly adhesive, such as an electrically conductive hydrogel.

9. The assembly according to any of the claims 1-8, wherein said cover comprises a flexible sheet and said first releasable attachment means are constituted by a peelable adhesive such that said flexible sheet may be manually peeled away from said container.

10. The assembly according to any of the claims 1-9, wherein said covering means is constituted by a plastics material foil, such as a PE, PP, PVC foil, an aluminium foil, a combination thereof or preferably a PE-Al-PE laminate.

11. The assembly according to any of the claims 1-10, wherein said first releasable attachment means is constituted by heat welding.

12. The assembly according to any of the claims 1-11, wherein said container is made from a plastic material such as PE, PP pr PVC, preferably a transparent or translucent material.

13. The assembly according to any of the claims 1-12, wherein said sensor defines a gripping part.

14. The assembly according to any of the claims 1-13, wherein said sensor comprises a woven, breathable cloth, a medical grade polyethylene foam and a sliver, silver chloride, tin chloride or graphite sensing element.

15. A set of storage and dispensing assemblies for individually storing and dispensing electrodes,
each of said electrodes devices comprising a sensor, a connector for connecting said sensor to a monitoring or measuring means and a electrically conductive wire for connecting said sensor to said connector, and
each of said assemblies comprising a container for receiving a respective electrode device and having a dispensing aperture closed by a cover attached to said container by means of first releasable attachment means, said sensor being realisably attached to a surface of said cover facing the interior of said container by means of second releasable attachment means.

16. The set of storage and dispensing assemblies according to claim 15, wherein each of said assemblies further comprises any of the features of the combination according to any of the claims 2-14.

17. A method for providing storage and dispensing of an electrode device from a storage and dispensing assembly, comprising the steps of:
providing said electrode device,
said electrode device comprising a sensor, a connector for connecting said sensor to a monitoring or measuring means and a electrically conductive wire for connecting said sensor to said connector,
providing an assembly, said assembly comprising a container for receiving said electrode device and having a dispensing aperture closed by a cover attached to said container by means of first releasable attachment means,
arranging said electrode device in said assembly, and
realisably attaching said sensor to a surface of said cover facing the interior of said container by means of second releasable attachment means.

18. The method according to claim 17, wherein said container defines cavity and a flange extending outward from said cavity, the method further comprising:
providing a circumferential groove formed in said flange, and
positioning said wire within said groove.

19. The method according to claim 18, further comprising:
providing a holder within said cavity, and
positioning said connector in said holder.

20. The method according to any of the claims 17-19, further comprising:
providing a gripping part in said cover for allowing a person to at least partly separate said cover from said container.

21. The method according to any of the claims 17-20, further comprising:
providing a gripping part at said sensor so as to allow a person to detach said sensor from said cover.

22. A method for providing storage and dispensing of a set of electrode devices from a set of storage and dispensing assemblies,
arranging each of said electrode devices in a specific assembly,
each of said electrode devices comprising a sensor, a connector for connecting said sensor to a monitoring or measuring means and a electrically conductive wire for connecting said sensor to said connector,
each of said assemblies comprising a container for receiving said electrode device and having a dispensing aperture closed by a cover attached to said container by means of first releasable attachment means, and
realisably attaching each of said sensors to a surface of a specific cover facing the interior of a specific container by means of second releasable attachment means.

23. The method according to claim 22, wherein said cover is constituted by a set of covers,
removing a specific cover of said set for exposing a specific electrode.

24. The method according to claim 22, wherein said cover is constituted by a unitary cover,
removing a specific cover for exposing a single specific electrode device and/or
removing said cover for exposing said set of electrode devices.

25. The method according to any of the claims 22-24, wherein said set of storage and dispensing assemblies and/or said set of electrode devices includes any of the features of the combination according to any of the claims 2-15 and/or said method including any of the steps of the method according to any of the claims 18-21.
